(19) Europäisches Patentamt

European Patent Office

Office européen des brevets

(11) EP 0 795 315 A2

# (12) EUROPÄISCHE PATENTANMELDUNG

(43) Veröffentlichungstag:
17.09.1997 Patentblatt 1997/38

(51) Int. Cl.$^6$: **A61K 7/42**, C12P 7/62

(21) Anmeldenummer: 97101186.1

(22) Anmeldetag: 27.01.1997

(84) Benannte Vertragsstaaten:
CH DE ES FR GB IT LI NL

(30) Priorität: 08.02.1996 DE 19604580

(71) Anmelder: HAARMANN & REIMER GMBH
D-37601 Holzminden (DE)

(72) Erfinder:
• Gatfield, Ian, Dr.
37671 Höxter (DE)
• Hall, Rüdiger
37603 Holzminden (DE)
• Kaiser, Reinhard, Dr.
37603 Holzminden (DE)
• Langner, Roland
37639 Bevern (DE)
• Surburg, Horst, Dr.
37603 Holzminden (DE)

(74) Vertreter: Petrovicki, Wolfgang, Dr.
Bayer AG
Konzernbereich RP
Patente und Lizenzen
51368 Leverkusen (DE)

(54) **Natürlicher p-Methoxyzimtsäureisoamylester, Verfahren zur natürlichen Herstellung höherer p-Methoxyzimtsäureester und ihre Verwendung als UV-Lichtschutzfilter**

(57) p-Methoxyzimtsäureisoamylester ist Inhaltsstoff einer Pflanzenwurzel und damit ein natürliches Produkt. Aus natürlichem p-Methoxyzimtsäureethylester können durch enzymatische Umesterung mit natürlichen Alkoholen höhere p-Methoxyzimtsäureester hergestellt werden.

EP 0 795 315 A2

**Beschreibung**

Die Erfindung betrifft natürlichen p-Methoxyzimtsäureisoamylester (nach-folgend pMZI), die biotechnologische Herstellung von höheren p-Methoxy-zimtsäurestern aus natürlichem p-Methoxyzimtsäureethylester (nachfolgend: pMZE), im besonderen die Herstellung von natürlichem pMZI, und die Verwendung dieser Verbindungen als UV-B-Licht-schutzfilter, vornehmlich in Sonnenschutzmitteln.

pMZI zahlt zu den wenigen Verbindungen, die im sogenannten UV-B-Bereich (280 bis 320 nm Wellenlänge) absorbieren und in vielen Ländern für den Einsatz in kosmetischen und pharmazeutischen Sonnenschutz-präparaten zugelassen sind. pMZI ist synthetisch gut zugänglich; natürliche Quellen für seine Gewinnung oder natürliche Verfahren zu seiner Herstellung waren bislang nicht bekannt.

In naturkosmetischen Produkten, die immer mehr an Bedeutung gewinnen, dürfen jedoch nur Substanzen verwendet werden, die ausschließlich auf Naturstoffen basieren [vgl. Seifen, Öle, Fette, Wachse 120(1), 24 (1994)]. Gemaß dieser Definition gibt es jedoch keine natürlichen Lichtschutz-verbindungen, die in der Anlage 7 der Kosmetikverordnung der BRD (entspricht EG-Richtlinie) als UV-Filter zugelassen sind.

Demnach war also eine Aufgabe der Erfindung die Bereitstellung natürlicher und natürlich hergestellter UV-B-Licht-schutzfilter.

pMZE ist Hauptbestandteil des etherischen Öls, das aus der Wurzel der in Indien beheimateten und in Südostasien (Südchina, Malaysia, Indonesien) für Würz- und medizinische Zwecke kultivierten Pflanze "Kaempferia galanga L." (malaysisch: "Kentjoor" oder "Kencur"; sundanesisch: "Tjeekor" oder "Chekur") gewonnen werden kann; vgl. Flavour and Fragrance Journal 7 (1992), 263-266. Gemäß dieser Literaturstelle beträgt der Gehalt im Öl ca. 52 Gew.-%, die durch Wasserdampfdestillation gewonnene Ausbeute an etherischem Öl ca. 0,8 Gew.-% (lt. Gildemeister/Hoffmann, Die Ätherischen Öle, Bd. IV, S. 481 kann die Ölausbeute auch bis zu 4 Gew.-%, bezogen auf getrocknete Wurzeln, betragen).

Wir haben nun überraschenderweise festgestellt, daß ein mit organischem Lösungsmittel gewonnener Kaempferia-galanga-Extrakt neben 50 bis 75 Gew.-% pMZE nicht nur 20 bis 25 Gew.-% Pentadecan und 3 bis 10 Gew.-% Zimtsäureethylester, sondern auch geringe Mengen (0,1 bis 0,2 Gew.-%) pMZI enthält. Damit ist zum ersten Mal der Beleg erbracht, daß pMZI auch ein natürliches Produkt ist.

Bevorzugte Lösungsmittel für die Extraktion sind bespielsweise Ethanol, Isopropanol, Aceton, Butanon, Toluol, Essigsäurethyl- oder -butylester und Gemische dieser Verbindungen. Die durch Lösungsmittelextraktion gewonnenen Kaempferia-galanga-Extrakte bestehen fast ausschließlich aus flüchtigen Verbindungen. Dies ist ungewöhnlich, da Extrakte im allgemeinen auch hohe Anteile an nicht verdampfbaren Materialien enthalten. Die durch Extraktion erhältliche Ausbeute an etherischem Öl beläuft sich auf 3 bis 5 Gew.-%, bezogen auf frische Wurzeln, oder 5 bis 20 Gew.-%, bezogen auf getrocknete Wurzeln. Gegenüber anderen natürlichen UV-B-Filtern, die häufig eine starke Färbung und/oder einen starken und damit störenden Eigengeruch besitzen, ist der aus Kaempferia-galanga-Extrakt gewonnene natürliche pMZE absolut rein. Die Gewinnung von pMZE aus dem Extrakt kann sowohl durch Destillation als auch durch Kristallisation erfolgen.

pMZE aus Kaempferia-galanga-Wurzeln eignet sich aufgrund seiner Ab-sorptionseigenschaften als natürlicher UV-B-Lichtschutzfilter. Weil aber in der Kosmetik wegen günstigerer anwendungstechnischer und dermatologi-scher Eigenschalten bevorzugt pMZ-Ester höherer Alkohole, z.B. der Isoamylester (= 3-Methylbutylester), verwendet werden, haben wir nach Wegen gesucht, diese auf Basis von p-MZE herzustellen.

Wir haben gefunden, daß sich pMZE mit natürlichen Alkoholen, wie sie beispielsweise bei der alkoholischen Gärung entstehen (z.B. Propanol, Isobutanol, Butanol, 2-Methylbutanol, 3-Methylbutanol = Isoamylakohol und Hexanol), in Gegenwart natürlicher Umesterungskatalysatoren (Enzyme) zu den entsprechenden höheren pMZ-Estern umestern lassen. Der Begriff "höher" im Sinne dieser Erfindung umfaßt 3 bis 6 C-Atome. Bei Verwendung von Isoamylalkohol darf der resultierende pMZI dann als natürlich bezeichnet werden. Die anderen natürlich herstellbaren Ester stellen wertvolle Lichtschutzfilter dar; sie sind potentielle natürliche Lichtschutzfilter für den Fall, daß der Nachweis als Naturstoff gelingt.

Geeignete Enzyme umfassen Lipasen und Esterasen, wie z.B. Esterase 30 000 aus Mucor miehei (Gist-Brocades oder Biocatalysts, UK), Lipase B aus Candida cylindracea (Biocatalysts, UK), immobilisierte Lipase von Candida antarctica (Novozym 435, Novo) oder aus Aspergillus oryzae (SP 524 und SP 525, Novo). Die Umesterung wird vorzugsweise in Gegenwart eines unter Reaktionsbedingungen inerten organischen Lösungsmittels, beispielsweise eines Kohlenwasserstoffs, wie Hexan, vorgenommen.

Die Umesterung wird vorzugsweise bei Temperaturen von 5 bis 35, insbesondere 40 bis 60°C durchgeführt. Der Reaktionsfortschritt kann durch geeignete Methoden, beispielsweise mittels Gaschromatographie, verfolgt werden. Die Reaktion kann abgebrochen werden, wenn der gewünschte Umsatz erreicht ist. Im allgemeinen ist dies nach 3 Stunden bis 30 Tagen der Fall.

Der so gewonnene pMZ-Ester kann durch übliche Methoden, wie beispielsweise Destillation, Chromatographie oder Kristallisation, von den Ausgangs- und Nebenprodukten abgetrennt werden.

"Natürlich" bzw. "natürlich hergestellte" pMZ-Ester im Sinne der Erfindung sind solche, deren pMZ-Säurereste folgende Isotopenwerte aufweisen (gemessen an aus den Estern durch Verseifung gewonnener pMZ-Säure):
$^{14}$C-Aktivität a$^{14}$: über 50 %, wobei gilt:

$$a^{14} = \text{Aktivität}_{Probe} / \text{Aktivität}_{Standard} \times 100 \, [\%].$$

Die Standardaktivität ergibt sich derzeit aus 0,95 x Aktivität Oxalsäure von 1950 (U.S. National Bureau of Standards)
$^{13}$C-Aktivität $\delta^{13}$C: über -32 ‰, wobei gilt:

$$\delta^{13}C = (([^{13}C]/[^{12}C])_{Probe} - ([^{13}C]/[^{12}C])_{PDP}) / ([^{13}C]/[^{12}C])_{PDB}) \times 1000 \, [‰].$$

PDB =     Standard Pee Dee Belemnite.

Deuterium = Aktivität $\delta^2$H: unter - 60 ‰, wobei gilt:

$$\delta^2H = (((^2H/^1H)_{Probe} / (^2H/^1H)_{VSMOW}) -1) \times 1000 \, [‰].$$

VSMOW = Vienna Standard Mean Ocean Water.

Gegenstand der Erfindung ist also natürlicher pMZI, ein Verfahren zur natürlichen Herstellung höherer pMZ-Ester durch enzymatische Umesterung von natürlichem pMZE mit natürlichen Alkoholen (und hierbei besonders die Herstellung von natürlichem pMZI).

Das erfindungsgemäße Verfahren hat den Vorteil, daß mit dem so gewonnenen pMZI ein bewährter und zugelassener UV-Lichtschutzfilter nicht mehr nur auf synthetischem Weg hergestellt werden kann, sondern jetzt auch wegen seiner Identifizierung als Naturstoff und seiner Gewinnung aus natürlichen Ausgangsmaterialien unter natürlichen Reaktionsbedingungen als Naturprodukt, z.B. für den Einsatz in Naturkosmetika, zur Verfügung steht.

Weiterer Gegenstand der Erfindung ist also die Verwendung von natürlichen und natürlich hergestellen höheren pMZ-Estern als UV-Lichtschutzfilter in kosmetischen bzw. pharmazeutischen Sonnenschutzpräparaten.

Die natürlichen bzw. die natürlich hergestellten pMZ-Ester werden in aller Regel in Mengen verwendet, die den Durchtritt der UV-Strahlen durch den aufgetragenen Film der Zubereitung verhindern. Dies ist dann der Fall, wenn die kosmetischen bzw. pharmazeutischen Zubereitungen 0,5 bis 15, vorzugsweise 1 bis 10, insbesondere 3 bis 8 Gew.-%, bezogen auf das Gesamtgewicht der Zubereitung, pMZ-Ester enthalten.

Die natürliche oder natürlich hergestellte pMZ-Ester enthaltenden Zube-reitungen können zum Schutz der Haut und der Haare - insbesondere durch Dauerwelle, Färbung und Bleichung bereits vorgeschädigter Haare - vor UV-Bestrahlung verwendet werden. Diese zum Schutz der Haut vor der UV-Strahlung dienenden kosmetischen oder pharmazeutischen Zubereitungen können in den üblicherweise verwendeten Anwendungsformen vorliegen, d.h. als Öl-in-Wasser- oder Wasser-in-Öl-Emulsion, als Milch, Lotion oder Creme, wäßrig oder wäßrig-alkoholisches Gel oder Lotion, Aerosol, Hydro-dispersions-Gel (emulgatorfrei) oder jegliche andere übliche kosmetische oder dermatologische Zubereitung. Für den Schutz der Haare vor UV-Strahlen werden bevorzugt Zubereitungen als Shampoo, Spülung, Kur, Gel, Lotion, Spray oder Creme verwendet. Die kosmetischen und dermatologischen Zubereitungen können die in diesen Mitteln üblicherweise verwendeten Bestandteile wie z.B. Emulgatoren, grenzflächenaktive Verbindungen, Lanolin, Vaseline, Wasser, Triglyceride von Fettsäuren, Polyethylenglykole, Fettalkohole, ethoxylierte Fettalkohole, Fettsäureester (z.B. Isopropyl-palmitat, Isooctylstearat, Adipinsäure-diisopropylester usw.), natürliche oder synthetische Öle oder Wachse, Pigmente (z.B. Titandioxid, Zinkoxid, Perlglanzpigmente, Farbpigmente), Verdickungsmittel (z.B. Hydroxyethylcellulose, Bentonit usw.), Konservierungsstoffe, Feuchtigkeitsmittel, Vitamine, Siliconöle, Glycerin, Ethylalkohol, Parfumöle enthalten. Die erfindungsgemäß zu verwendenden Verbindungen können als einzige UV-Absorber in den entsprechenden Zubereitungen eingesetzt werden; man kann sie jedoch auch in Kombination mit anderen UV-Absorbern einsetzen, insbesondere mit UV-A-Absorbern zur Erzielung einer UV-A+B-Breitband-absorption. Beispiele für solche Verbindungen umfassen:

    p-Aminobenzoesäure
    p-Aminobenzoesäureethylester, ethoxyliert (25 Mol)
    p-Dimethylaminobenzoesäure-2-ethylhexylester
    p-Aminobenzoesäureethylester, propoxyliert (2 Mol)
    p-Aminobenzoesäureglycerinester
    Salicylsäure-homomenthylester
    Salicylsäure-2-ethylhexylester
    Triethanolaminsalicylat
    4-Isopropylbenzylsalicylat

Anthranilsäurementhylester

Diisopropylzimtsäureethylester

p-Methoxyzimtsäure-2-ethylhexylester

2-Hydroxy-4-methoxybenzophenon

2-Hydroxy-4-methoxybenzophenon-5-sulfonsäure

Dihydroxy-4-methoxybenzophenon

2,4-Dihydroxybenzophenon

Tetrahydroxybenzophenon

2,2'-Dihydroxy-4,4'-dimethoxybenzophenon

2-Hydroxy-4-n-octoxybenzophenon

2-Hydroxy-4-methoxy-4'-methylbenzophenon

a-(2 -Oxoborn-3-yliden)-tolyl-4-sulfonsäure und deren Salze

3-(4'-Methylbenzyliden)-d,l-campher

3-Benzyliden-d,l-campher

4-Isopropyldibenzoylmethan

2,4,6-Trianilino-(p-carbo-2'-ethylhexyl1'-oxy)- 1,3 ,5-triazin

1 ,4-Phenylen-bis-(2-benzimidazolyl-5 ,7-disulfonsäure)

Diisopropylzimtsäuremethylester

p-Methoxyzimtsäureisoamylester, synthetisch

p-Methoxyzimtsäure-diethanolaminsalz

p-Methoxyzimtsäure-isopropylester

2-Ethylhexyl-2-cyano-3,3-diphenylacrylat

Ethyl-2-cyano-3,3-diphenylacrylat

2-Phenylbenzimidazolsulfonsäure und deren Salze

N,N,N-Trimethyl-4-(2-oxoborn-3-ylidenmethyl)aniliniummethylsulfat

Tetraphthalyliden-dibornansulfonsäure und deren Salze

4-tert.-Butyl-4'-methoxy-dibenzoylmethan

$\beta$-Imidazol-4(5)-acrylsäure (Urocaninsäure)

Die Prozentangaben der nachfolgenden Beispiele beziehen sich auf das Gewicht; Teile sind Gewichtsteile.

### Beispiele

### Beispiel 1

**Gewinnung von Kaempferia-galanga-Extrakt bzw. natürlichem p-Methoxyzimtsäureethylester**

1 kg frische geschnittene Kaempferia-galanga-Wurzeln aus Indonesien wurden dreimal mit jeweils 1,5 l Ethanol für mehrere Stunden ausgekocht. Die ethanolischen Extrakte wurden abgegossen, vereinigt und eingedampft. Als Rückstand verblieb eine hellbraune teilweise kristallisierte Masse; Ausbeute:ca. 45 - 55 g. Laut GC (60m DB-1) besteht das erhaltene Produkt zu ca. 60 - 75 % aus p-Methoxy-zimtsäureethylester, zu 15 - 25 % aus n-Pentadecan und zu 5 - 10 % aus Zimtsäureethylester. Eine eingehendere Analyse der Inhaltstoffe durch GC-MS-Kopplung zeigte eindeutig ebenfalls die Anwesenheit von 0,1 - 0,2 % p-Methoxyzimtsäureisoamylester. Der erhaltene Extrakt kann als solcher weiterverarbeitet werden, bietet aber auch die Möglichkeit, den p-Methoxyzimtsäureethylester in reiner Form durch Destillation oder durch Kristallisation, z.B. durch Aufnehmen in Essigester und Ausfällen mit Hexan, zu gewinnen; Ausbeute, bezogen auf eingesetzten Extrakt: ca. 70 %, Schmp. 48,2 °C; Reinheit nach GC (60m DB-1): 99,4 %; UV: $\lambda_{max}$ 308 nm, $E_{1/1}$ 1179.

Durch alkalische Verseifung aus dem pMZE erhaltene p-MZ-Säure ergab folgende Werte:

$^{14}a =$      117 %

$\delta^{13}C =$      -28.86 ‰

$\delta^2H =$      -68 ‰

Aus Anisaldehyd hergestellte pMZ-Säure ($^{®}$Neo Heliopan E 1000, Handelsprodukt der Firma Haarmann & Reimer GmbH, Holzminden) besaß dagegen folgende Eigenschaften:

$^{14}a =$      < 0,5 %

$\delta^{13}C =$      -35,67 ‰

$\delta^2H =$      54 ‰

### Beispiel 2

**Enzymatische Umsetzung von natürlichem p-Methoxyzimtsäure-ethylester (pMZE) und natürlichem Gärungsisoamylalkohol mit Esterase 30.000 aus Mucor mihei (Gist-Brocades):**

pMZE (0,42 g, 2 mMol) und Isoamylalkohol (1,76 g, 20 mMol) wurden in 30 ml n-Hexan gelöst, mit 1,6 g Esterase versetzt und bei 30°C im geschlossenen Kolben geschüttelt. Nach 7 Tagen wurde per GC eine Umsetzung von 24,8 % (Verhältnis pMZI zu pMZE) ermittelt.

### Beispiel 3

**Enzymatische Umsetzung von natürlichem p-Methoxyzimtsäure-ethylester (pMZE) und natürlichem Gärungsisoamylalkohol mit Esterase aus Mucor mihei (Biocatalysts, UK):**

pMZE (2,1 g, 10 mMol) und Isoamylalkohol (17,6 g, 200 mMol) wurden in 2 ml n-Hexan gelöst, mit 2 g Esterase versetzt und bei 45°C im geschlossenen Kolben geschüttelt. Nach 7 Tagen wurde per GC eine Umsetzung von 29,8 % (Verhältnis pMZI zu pMZE) ermittelt.

### Beispiel 4

**Enzymatische Umsetzung von natürlichem p-Methoxyzimtsäure-ethylester (pMZE) und natürlichem Gärungsisoamylalkohol mit Lipase B aus Candida cylindracea (Biocatalysts, UK):**

pMZE (0,42 g, 2 mMol) und Isoamylalkohol (1,76 g, 20 mMol) wurden in 30 ml n-Hexan gelöst, mit 1,6 g Lipase versetzt und bei 30°C im geschlossenen Kolben geschüttelt. Nach 6 Tagen wurde per GC eine Umsetzung von 29 % (Verhältnis pMZI zu pMZE) ermittelt.

### Beispiel 5

**Enzymatische Umsetzung von natürlichem p-Methoxyzimtsäure-ethylester (pMZE) und natürlichem Gärungsisoamylalkohol mit Lipase aus Candida antarctica (Novozym 435, Novo):**

pMZE (0,42 g, 2 mMol) und Isoamylalkohol (1,76 g, 20 mMol) wurden in 15 ml n-Hexan gelöst, mit 2 g Lipase versetzt und bei 30°C im geschlossenen Kolben geschüttelt. Nach 7 Tagen wurde per GC eine Umsetzung von 91 % (Verhältnis pMZI zu pMZE) ermittelt.

### Beispiel 6

**Enzymatische Umsetzung von natürlichem p-Methoxyzimtsäure-ethylester (pMZE) und natürlichem Gärungsisoamylalkohol mit Lipase aus Candida antarctica (Novozym 435, Novo):**

pMZE (8,4 g, 40 mMol) und Isoamylalkohol (17,6 g, 200 mMol) wurden in 10 ml n-Hexan gelöst, mit 1 g Lipase versetzt und bei 45°C im geschlossenen Kolben geschüttelt. Nach 7 Tagen wurde per GC eine Umsetzung von 62,1 %, nach 10 Tagen eine Umsetzung von 74,5 % (Verhältnis pMZI zu pMZE) ermittelt.

### Beispiel 7

**Enzymatische Umsetzung von natürlichem p-Methoxyzimtsäure-ethylester (pMZE) und natürlichem Gärungsisoamylalkohol mit Lipase aus Candida antarctica (Novozym 435, Novo):**

pMZE (8,4 g, 40 mMol) und Isoamylalkohol (17,6 g, 200 mMol) wurden in 10 ml n-Hexan gelöst, mit 2 g Lipase versetzt und bei 45°C im geschlossenen Kolben geschüttelt. Nach 7 Tagen wurde per GC eine Umsetzung von 72,5 %, nach 10 Tagen eine Umsetzung von 78,5 % (Verhältnis pMZI zu pMZE) ermittelt.

**Beispiel 8**

**Enzymatische Umsetzung von natürlichem p-Methoxyzimtsäure-ethylester (pMZE) und natürlichem n-Propanol aus Fuselöl mit Lipase aus Candida antarctica (Novozym 435, Novo):**

pMZE (2,1 g, 10 mMol) und n-Propanol (12,2 g, 200 mMol) wurden in 2 ml n-Hexan gelöst, mit 2 g Lipase versetzt und bei 45°C im geschlossenen Kol-ben geschüttelt. Nach 4 Tagen wurde per GC eine Umsetzung von 90,4 %, nach 8 Tagen eine Umsetzung von 95,3 % (Verhältnis pMZ-propylester zu pMZE) ermittelt.

**Beispiel 9**

**Enzymatische Umsetzung von natürlichem p-Methoxyzimtsäure-ethylester (pMZE) und natürlichem n-Butanol aus Fuselöl mit Lipase aus Candida antarctica (Novozym 435, Novo):**

pMZE (2,1 g, 10 mMol) und n-Butanol (14,8 g, 200 mMol) wurden in 2 ml n-Hexan gelöst, mit 2 g Lipase versetzt und bei 45°C im geschlossenen Kolben geschüttelt. Nach 4 Tagen wurde per GC eine Umsetzung von 76,4 %, nach 8 Tagen eine Umsetzung von 92,4 % (Verhältnis pMZ-butylester zu pMZE) ermittelt.

**Beispiel 10**

**Enzymatische Umsetzung von natürlichem p-Methoxyzimtsäure-ethylester (pMZE) und natürlichem Isobutanol aus Fuselöl mit Lipase aus Candida antarctica (Novozym 435, Novo):**

pMZE (2,1 g, 10 mMol) und Isobutanol (14,8 g, 200 mMol) wurden in 2 ml n-Hexan gelöst, mit 2 g Lipase versetzt und bei 45°C im geschlossenen Kolben geschüttelt. Nach 4 Tagen wurde per GC eine Umsetzung von 93,6 %, nach 8 Tagen eine Umsetzung von 95,7 % (Verhältnis pMZ-isobutylester zu pMZE) ermittelt.

**Beispiel 11**

**Enzymatische Umsetzung von natürlichem p-Methoxyzimtsäure-ethylester (pMZE) und natürlichem 2-Methylbutanol aus Fuselöl mit Lipase aus Candida antarctica (Novozym 435, Novo):**

pMZE (2,1 g, 10 mMol) und 2-Methylbutanol (17,6 g, 200 mMol) wurden in 2 ml n-Hexan gelöst, mit 2 g Lipase versetzt und bei 45°C im geschlossenen Kolben geschüttelt. Nach 4 Tagen wurde per GC eine Umsetzung von 94,9 %, nach 8 Tagen eine Umsetzung von 96,7% (Verhältnis pMZ-2-methyl-butylester zu pMZE) ermittelt.

**Beispiel 12**

**Enzymatische Umsetzung von natürlichem p-Methoxyzimtsäure-ethylester (pMZE) und natürlichem n-Hexanol aus Fuselöl mit Lipase aus Candida antarctica (Novozym 435, Novo):**

pMZE (2,1 g, 10 mMol) und n-Hexanol (20,4 g, 200 mMol) wurden in 2ml n-Hexan gelöst, mit 2 g Lipase versetzt und bei 45°C im geschlossenen Kol-ben geschüttelt. Nach 4 Tagen wurde per GC eine Umsetzung von 92,5%, nach 8 Tagen eine Umsetzung von 94,5 % (Verhältnis pMZ-hexylester zu pMZE) ermittelt.

**Beispiel 13**

**Enzymatische Umsetzung von natürlichem p-Methoxyzimtsäure-ethylester (pMZE) und natürlichem Gärungsisoamylalkohol mit Lipase aus Aspergillus oryzae (SP 524, Novo):**

pMZE (2,1 g, 10 mMol) und Isoamylalkohol (17,6 g, 200 mMol) wurden in 2 ml n-Hexan gelöst, mit 2 g Lipase versetzt und bei 45°C im geschlossenen Kolben geschüttelt. Nach 4 Tagen wurde per GC eine Umsetzung von 74,5 %, nach 10 Tagen eine Umsetzung von 91,4 % (Verhältnis pMZI zu pMZE) ermittelt.

### Beispiel 14

**Enzymatische Umsetzung von natürlichem p-Methoxyzimtsäure-ethylester (pMZE) und natürlichem Gärungsisoamylalkohol mit Lipase aus Aspergillus oryzae (SP 525, Novo):**

pMZE (2,1 g, 10 mMol) und Isoamylalkohol (17,6 g, 200 mMol) wurden in 2 ml n-Hexan gelöst, mit 2 g Lipase versetz: und bei 45°C im geschlossenen Kolben geschüttelt. Nach 4 Tagen wurde per GC eine Umsetzung von 52,7 %, nach 10 Tagen eine Umsetzung von 78,8 % (Verhältnis pMZI zu pMZE) ermittelt.

### Beispiel 15

**Enzymatische Umsetzung von natürlichen p-Methoxyzimtsäureethylester(pMZE) enthaltendem Kaempferia-galanga-Extrakt und natürlichem Gärungsisoamylalkohol mit Lipase aus Candida antarctica (Novozym 435, Novo) und Isolierung von natürlichem p-Methoxyzimtsäureisoamylester (pMZI):**

23 g Kaempferia-galanga-Extrakt (entsprechend 14,9 g bzw. 70 mMol pMZE bei einem Gehalt von 65 %) und Isoamylalkohol (123 g, 1400 mMol) wurden in 14 ml n-Hexan gelöst, mit 7 g Lipase versetzt und bei 45°C im geschlossenen Kolben geschüttelt. Nach 7 Tagen wurde per GC eine Umsetzung von 72,5 %, nach 10 Tagen eine Umsetzung von 86,6 % (Verhältnis pMZI:pMZE) ermittelt. Nach Abfiltrieren des Enzyms wurde der Isoamylalkohol im Wasserstrahlvakuum über ein 10 cm-Destillationskolonne abdestilliert. Der Rück-stand wurde über eine 10 cm-Vigreux-Kolonne fraktioniert. Bei einem Siedepunkt von 125-135°C/ 0,4 mbar wurden 13,9 einer Fraktion erhalten, die zu 98,4 % aus natürlichem pMZI bestand.

UV: $\lambda_{max\ 308\ nm,\ E\ 1/1}$ 977.

**Beispiel 16**

**Herstellung einer Sonnenschutzlotion (O/W) unter Verwendung von natürlichem p-Methoxyzimtsäureisoamylester (pMZI):**

| Bestandteile | % |
|---|---|
| A) | |
| Arlatone 983 S | 1,50 |
| Brij 76 | 1,00 |
| Lanette O | 1,20 |
| Paraffinöl 65 cp | 3,00 |
| Cegesoft C 24 | 4,00 |
| Eutanol G | 4,50 |
| Baysilonöl M 10 | 1,00 |
| **pMZI natürlich** | 5,00 |
| B) | |
| Wasser, dest. | 49,50 |
| 1,2-Propylenglykol | 2,00 |
| Phenonip | 0,50 |
| C) | |
| Wasser, dest. | 25,00 |
| Carbopol 934 | 0,30 |
| Natriumhydroxid, 10%ig in Wasser | 1,10 |
| D) Parfümöl | 0,40 |

Herstellvorschrift: 1.Teil A bei ca. 80° C aufschmelzen. 2.Teil B auf ca. 90 °C erhitzen und unter Rühren zu Teil A geben, auf Raumtemperatur abrühren.

3. Von Teil C Carbopol klumpenfrei in Wasser dispergieren, mit Natriumhydroxid-Lösung zu einem Gel neutralisieren, bei ca. 60° C zu Teil A/B geben. 4. Bei ca. 30°C die Emulsion mit Teil D parfümieren, pH-Wert kontrollieren (ca. 7).

Von der auf diese Art hergestellten Sonnenschutzlotion wurde ein in-vitro-Sonnenschutzfaktor nach der Methode von B.L. Diffey und J.Robson [J. Soc. Cosmet. Chem. 40, 127 (1989)] mit 9,1 bestimmt.

**Beispiel 17**

**Herstellung einer Sonnenschutzlotion (O/W) unter Verwendung von natürlichem p-Methoxyzimtsäureisoamylester (pMZI):**

| Bestandteile | % |
|---|---|
| A) | |
| Cutina FS 45 | 2,00 |
| Eumulgin B 1 | 0,25 |
| Eumulgin B 2 | 0,25 |
| Cutina MD | 2,00 |
| Lanette O | 2,80 |
| Myritol 318 | 3,00 |
| Paraffinöl 65 cp | 3,50 |
| Abil 100 | 0,50 |
| Copherol 1250 / V | 1,00 |
| Arosol | 0,80 |
| Solbrol P | 0,10 |
| Neo Heliopan, Typ AV / p-Methoxyzimtsäureisooctylester | 4,00 |
| **pMZI natürlich** | 4,00 |
| Neo Heliopan, Typ BB / 2-Hydroxy-4-methoxybenzophenon | 2,00 |
| B) | |
| Wasser, dest. | 38,45 |
| Carbopol 980 | 0,30 |
| Natriumhydroxid, 10%ig in Wasser | 2,45 |
| 1,2-Propylenglykol | 2,00 |
| Solbrol M | 0,20 |
| Neo Heliopan, Typ Hydro, eingesetzt als 15%ige Lösung nach Neutralisation mit Natriumhydroxid/Phenylbenzimidazolsulfonsäure(entspricht Aktivsubstanz : 4,5 %) | 30,00 |
| C) | |
| Parfümöl | 0,40 |

Herstellvorschrift: 1. Teil A bei ca. 80° C aufschmelzen. 2. Von Teil B Carbopol klumpenfrei im Wasser dispergieren, mit Natronlauge neutralisieren, die restlichen Inhaltsstoffe zugeben und auf ca. 95 °C erhitzen. Teil B unter Rühren zu Teil A geben und auf Raumtemperatur abrühren. 3. Bei ca. 30 ° C Teil C zugeben. pH-Wert überprüfen (7,0 - 7,5).

**Beispiel 18**

**Herstellung einer Sonnenschutz-Creme (O/W) unter Verwendung von natürlichem p-Methoxyzimtsäureisoamylester (pMZI):**

| Bestandteile | % |
|---|---|
| A) | |
| Arlacel 165 | 3,00 |
| Eumulgin B 2 | 1,00 |
| Lanette O | 2,00 |
| Myritol 318 | 4,00 |
| Cetiol OE | 3,00 |
| Abil 100 | 1,00 |
| Betone Gel MIO | 3,00 |
| Phenonip | 0,20 |
| Cutina CBS | 2,00 |
| Neo Heliopan, Typ AV/p-Methoxyzimtsäureisooctylester | 5,00 |
| *pMZI natürlich* | 5,00 |
| Neo Heliopan, Typ MBC / 4-Methylbenzyliden-campher | 1,50 |
| Neo Heliopan, Typ OS / Octylsalicylat | 3,00 |
| B) | |
| Wasser, dest. | 31,70 |
| Veegum Ultra | 1,00 |
| Glycerin 86 % | 3,00 |
| Phenonip | 0,30 |
| Neo Heliopan, Typ Hydro, eingesetzt als 15%ige Lösung nach Neutralisation mit Natriumhydroxid (Phenylbenzimidazolsulfonsäure, entspricht Aktivsubstanz : 3,00 %) | 20,00 |
| Zink Oxide neutral H&R /Zinkoxid neutral | 10,00 |
| C) | |
| Parfumöl | 0,30 |

Herstellvorschrift: 1. Teil A bei ca. 80° C aufschmelzen. 2. Bestandteile von Teil B ohne Veegum und Zinkoxid neutral auf ca. 90° C erhitzen, dann Veegum und Zinkoxid neutral sorgfältig dispergieren. Teil B unter Rühren zu Teil A geben; auf Raumtemperatur abrühren. 3. Bei 30° C Teil C zugeben und anschließend homogenisieren; pH-Wert überprüfen (7,0-7,5).

**Beispiel 19**

**Herstellung eines emulgatorfreien Sonnenschutz-Hydrodispersionsgels unter Verwendung von natürlichem p-Methoxyzimtsäureisoamylester (pMZI):**

| Bestandteile: | % |
|---|---|
| A) | |
| Wasser, dest. | 64,50 |
| Carbopol 1342 | 1,00 |
| Neutrol TE | 1,70 |
| B) | |
| Neo Heliopan, Typ Hydro, eingesetzt als 15%ige Lösung nach Neutralisation mit Natriumhydroxid (Phenylbenzimidazolsulfonsäure; entspricht 3 % Aktivsubstanz ) | 20,00 |
| Neo Heliopan, Typ AV / p-Methoxyzimtsäureisooctylester | 3,00 |
| **pMZI, natürlich** | 3,00 |
| Neo Heliopan, Typ BB / 2-Hydroxy-4-methoxybenzophenon | 1,00 |
| Isopropylmyristat | 3,00 |
| Baysilonöl PK 20 | 2,00 |
| Phenonip | 0,50 |
| Parfümöl | 0,30 |

Herstellvorschrift: 1. (Teil A) Carbopol klumpenfrei im Wasser dispergieren und mit Neutrol TE neutralisieren. 2. Neo Heliopan, Typ Hydro-Lösung (Teil B) in Teil A einführen. 3. Von Teil C Neo Heliopan, Typ BB in Heliopan, Typ AV und pMZI natürlich lösen. Die restlichen Bestandteile zugeben und Teil C unter Rühren zu Teil A/B geben. Anschließend homogenisieren (Kolloid-Mühle) und den pH-Wert kontrollieren (7,0-7,5).

**Beispiel 20**

**Herstellung eines Haarbalms mit Sonnenschutz unter Verwendung von natürlichem p-Methoxyzimtsäureisoamylester (pMZI):**

| Bestandteile | % |
|---|---|
| A) | |
| Wasser, dest. | 58,40 |
| Phenonip | 0,40 |
| D-Panthenol | 0,50 |
| 1,2-Propylenglykol | 5,00 |
| Trilon B fl. | 0,10 |
| Carbopol ETD 2001 | 0,50 |
| B) | |
| Wasser dest. | 25,00 |
| Natriumhydroxid, 10%ig in Wasser | 2,80 |
| Luviskol K 30 | 2,50 |
| C) | |
| **pMZI natürlich** | 4,00 |
| Neo Heliopan, Typ BB /2-Hydroxy-4-methoxybenzophenon | 0,60 |
| Parfümöl | 0,20 |

Herstellvorschrift :

1. Inhaltsstoffe von Teil A in Wasser lösen, Carbopol klumpenfrei dispergieren. 2. Inhaltsstoffe von Teil B im Wasser lösen, Teil B unter Rühren zu Teil A geben. 3. Von Teil C Neo Heliopan, Typ BB in pMZI, natürlich lösen und Parfümöl zugeben. Teil C unter Rühren zu Teil A/B geben. Anschließend homogenisieren (Kolloid-Mühle).

**Beispiel 21**

**Herstellung eines Haarsprays mit Sonnenschutz, Non-Aerosol-Typ, unter Verwendung von natürlichem p-Methoxyzimtsäureisoamylester**

| Bestandteile | % |
|---|---|
| A) | |
| Ethylalkohol | 50,00 |
| **pMZI natürlich** | 1,60 |
| Neo Heliopan, Typ BB /2-Hydroxy-4-methoxy-benzophenon | 0,40 |
| Cremophor RH 60 | 3,20 |
| Cremophor NP 14 | 1,80 |
| Parfümöl | 0,40 |
| B) | |
| Wasser, dest. | 42,40 |
| Dehyquart A | 0,20 |

Herstellvorschrift : 1. Inhaltsstoffe von Teil A im Ethylalkohol lösen.

2. Dehyquart A im Wasser lösen, Teil B zu Teil A geben und verrühren.

**Beispiel 22**

**Herstellung einer Sonnenschutzlotion (W/O) unter Verwendung von Kaempferia-galanga-Extrakt**

| Bestandteile | % |
|---|---|
| A) | |
| Arlacel 481 | 3,50 |
| Arlacel 989 | 1,50 |
| Eutanol G | 4, 00 |
| Isopropylisostearat | 5, 00 |
| Paraffinöl 65 cp | 4, 60 |
| Baysilonöl M 10 | 1. 00 |
| Vaseline Typ "Merkur" 1546 | 7,50 |
| **K-galanga-Extrakt** | 4, 00 |
| B) | |
| Wasser, dest. | 65, 40 |
| Phenonip | 0,50 |
| Glycerin 86 % | 2, 00 |
| Magnesiumsulfat | 0,50 |
| C) | |
| Parfümöl | 0, 50 |

Herstellvorschrift :

1. Teil A bei ca. 80° C aufschmelzen. 2. Teil B auf ca. 90° C erhitzen, langsam unter Rühren zu Teil A geben. Auf Raumtemperatur abrühren. 3. Die Emulsion bei ca. 35° C mit Teil C parfümieren und anschließend homogenisieren.

Von der auf diese Art hergestellten Sonnenschutzlotion wurde ein in-vitro-Sonnenschutzfaktor nach der Methode von B.L. Diffey und J.Robson [J. Soc. Cosmet. Chem. 40, 127 (1989)] mit 5,9 bestimmt.

**Beispiel 23**

**Herstellung einer Sonnenschutzlotion (O/W) unter Verwendung von Kaempferia-galanga-Extrakt**

| Bestandteile | % |
|---|---|
| A) | |
| Tegin M | 3,50 |
| Tagat S | 1,50 |
| Lanette O | 2, 70 |
| Paraffinöl 65 cp | 2,00 |
| Myrtol 318 | 3,00 |
| Arlamol HD | 2,00 |
| Abil 100 | 0,50 |
| Cetiol MM | 2,00 |
| Phenonip | 0,20 |
| Neo Heliopan, Typ 303 / Octocrylen | 3,00 |
| *K-galanga-Extrakt* | 3,00 |
| B) | |
| Wasser, dest. | 33,30 |
| Phenonip | 0,20 |
| Neo Heliopan, Typ Hydro, eingesetzt als 15%ige Lösung nach Neutralisation mit Natriumhydroxid/Phenylbenzimidazolsulfonsäure entsprechend 2,00 % Aktivsubstanz | 13,35 |
| 1, 2-Propylenglykol | 2,00 |
| C) | |
| Wasser dest. | 25,00 |
| Carbopol 934 | 0,40 |
| Natriumhydroxid, 10%ig in Wasser | 2,05 |
| D) | |
| Parfümöl | 0,30 |

Herstellvorschrift : 1. Teil A bei ca. 80° C aufschmelzen. 2. Teil B auf ca. 90° C erhitzen, unter Rühren zu Teil A geben, auf Raumtemperatur abrühren. 3. Von Teil C Carbopol klumpenfrei in Wasser dispergieren, mit Natriumhydroxid-Lösung zu einem Gel neutralisieren, bei ca. 60° C zu Teil A/B geben. 4. Bei ca. 30° C die Emulsion mit Teil D parfümieren; pH-Wert kontrollieren (7,0 - 7,5).

**Beispiel 24**

**Herstellung einer Sonnenschutz-Creme (W/O) unter Verwendung von Kaempferia-galanga-Extrakt**

| Bestandteile | % |
|---|---|
| A) | |
| Arlacel 481 | 3,50 |
| Arlacel 989 | 1,50 |
| Eutanol G | 2,50 |
| Isopropylisostearat | 2,50 |
| Paraffinöl 65 cp | 3,50 |
| *K-galanga-Extrakt* | 4,00 |
| Neo Heliopan, Typ AV / p-Methoxyzimtsäureisooctylester | 4,00 |
| Neo Heliopan, Typ BB / 2-Hydroxy-4-methoxybenzophenon | 1,00 |
| Baysilone Fluid M 10 | 1,00 |
| Vaseline Typ Merkur 1546 | 7,50 |
| Solbrol P | 0,08 |
| B) | |
| Wasser, dest. | 64,42 |
| Solbrol M | 0,20 |
| Glycerin 86 % | 2,00 |
| Magnesiumsulfat | 2,00 |
| C) | |
| Parfümöl | 0,30 |

Herstellvorschrift :

1.Teil A bei ca. 80° C aufschmelzen. 2. Teil B auf ca. 90°C erhitzen und langsam unter Rühren zu Teil A geben. Auf Raumtemperatur abrühren.

3. Die Emulsion mit Teil C bei ca. 35° C parfümieren, anschließend homogenisieren.

**Beispiel 25**

**Herstellung einer Sonnenschutzlotion (W/O) unter Verwendung von Kaempferia-galanga-Extrakt**

| Bestandteile | % |
|---|---|
| A) | |
| Lameform TGI | 4,00 |
| Dehymuls HR E 7 | 4,00 |
| Cetiol OE | 7,50 |
| Isopropylisostearat | 8,00 |
| Baysilone Fluid M 10 | 1,00 |
| Permulgin 3220 | 1,50 |
| Neo Heliopan, Typ AV / p-Methoxyzimtsäureisooctylester | 5,00 |
| *K-galanga-Extrakt* | 3,00 |
| Neo Heliopan, Typ MBC / 4-Methylbenzyliden-campher | 1,00 |
| Neo Heliopan, Typ BB / 2-Hydroxy-4-methoxy benzophenon | 1,50 |
| B) | |
| Wasser, dest. | 54,10 |
| Glycerin 86 % | 3,00 |
| Magnesiumsulfat | 0,50 |
| Phenonip | 0,50 |
| Zinc Oxide neutral H&R / Zinkoxid neutral | 5,00 |
| C) | |
| Parfümöl | 0,40 |

Herstellvorschrift: 1. Teil A bei ca. 80° C aufschmelzen. 2. Bestandteile von Teil B zusammengeben, auf ca. 90° C erhitzen und Zinkoxid neutral sorgfältig dispergieren. Teil B langsam unter Rühren zu Teil A geben. Auf Raumtemperatur abrühren. 3. Die Emulsion mit Teil C bei ca. 35°C parfümieren, anschließend homogenisieren.

**Beispiel 26**

**Herstellung eines Lip-Sticks mit Sonnenschutz unter Verwendung von Kaempferia-galanga-Extrakt**

| Bestandteile | % |
|---|---|
| A) | |
| Lunacera LBN | 55,00 |
| Neo Heliopan, Typ AV / p-Methoxyzimtsäureisooctylester | 4,00 |
| *K-galanga-Extrakt* | 4,00 |
| Neo Heliopan, Typ MBC / 4-Methylbenzylidencampher | 1,00 |
| Neo Heliopan, Typ BB / 2-Hydroxy-4-methoxybenzophenon | 1,00 |
| Zinc Oxide neutral H&R /Zinkoxid neutral | 5,00 |
| Myritol 318 | 7,00 |
| Eutanol G | 6,00 |
| Cetiol 86 8 | 5,00 |
| Cetiol MM | 5,00 |
| Jojobaöl | 1,00 |
| Tocopherolöl CLR | 0,50 |
| Rewopal PIB 1000 | 5,00 |
| Parfümöl | 0,50 |

Herstellvorschrift : Bestandteile einwiegen ohne Zinc Oxide neutral H&R und Parfümöl. Bei ca. 80° C aufschmelzen, das Zinc Oxide neutral H&R zugeben und das Produkt über einen Dreiwalzenstuhl geben. Das Produkt erneut aufschmelzen und nochmals über den Walzenstuhl geben. Das Produkt erneut aufschmelzen, parfümieren und in entsprechende Formen vergießen.

**Beispiel 27**

**Herstellung eines Haar-Shampoos mit Sonnenschutz unter Verwendung von Kaempferia-galanga-Extrakt**

| Bestandteile | % |
|---|---|
| A) | |
| Genapol LRO fl. | 18,00 |
| Texapon MG 3 | 36,00 |
| Lamepon S | 6,00 |
| *K-galanga-Extrakt* | 1,00 |
| Parfümöl | 0,60 |
| Phenonip | 0,50 |
| B) | |
| Wasser, dest. | 35,60 |
| Polymer JR 400 | 0,20 |
| Natriumchlorid | 1,00 |
| Natriumhydroxid, 10%ig in Wasser | 0,10 |
| D-Panthenol | 1,00 |

Herstellvorschrift : 1. Bestandteile von Teil A mischen. 2. Bestandteile von Teil B im Wasser lösen. 3.Teil B unter Rühren zu Teil A geben. Rühren bis homogenes Produkt entstanden ist und pH-Wert überprüfen (ca. 7).

**Beispiel 28**

**Herstellung eines Haaröls mit Sonnenschutz unter Verwendung von Kaempferia-galanga-Extrakt**

| Bestandteile | % |
|---|---|
| A) | |
| Paraffinöl 200 cp | 94,50 |
| Neo Heliopan, Typ AV / p-Methoxyzimtsäureisooctylester | 2,00 |
| *K-galanga-Extrakt* | 2,00 |
| Neo Heliopan, Typ BB / 2-Hydroxy-4-methoxybenzophenon | 1,00 |
| Parfümöl | 0,50 |

Herstellvorschrift : Alle Bestandteile vermischen.

**Anhang zu den Beispielen 16 bis 28**

Verwendete Komponenten (Bezugsquellen in Klammern):

Abil 100: Siliconöl, (8)

Arlacel 165: Glycerinstearat/Polyethylenglykol(MG 100)-stearat-Mischung, (4)

Arlacel 481: Glycerol Sorbitan Oleostearate, (4)

Arlacel 989: ethoxyliertes hydriertes Ricinusöl, (4)

Arlamol HD: Isohexadecan,(4)

Arlatone 983 S: Polyethylenglykol (MG5)-Glycerinstearat, (4)

Arosol: Phenoxyethanol, (1)

Baysilone Fluid M 10: Siliconöl, (5)

Baysilone Fluid PK 20: Siliconöl, (5)

Betone Gel MIO: Mineralöl und Quaternium-18 Hectorite, (11)

Brij 76: Stearylalkohol, verethert mit 10 Mol Ethylenoxid, (4)

Carbopol 934: Polyacrylsäure, (2)

Carbopol 954: Polyacrylsäure, (2)

Carbopol 980: Polyacrylsäure, (2)

Carbopol 1342: Polyacrylat, (2)

Carbopol ETD 2001: Acrylsäure-Copolymerisat, (2)

Cegesoft C 24: Ethylhexylpalmitat, (3)

Cetiol MM: Myristyl-myristat, (3)

Cetiol OE: Dicaprylether, (3)

Cetiol 868: Isooctylstearat, (3)

Copherol 1250: Vitamin-E-Acetat, (3)

Cremophor NP 14: mit 14 Mol Ethylenoxid verethertes Nonylphenol, (6)

Cremophor RH 60: ethoxygliertes gehärtetes Ricinusöl, (6)

Cutina CBS: Glycerinstearat, Cetyl-/Stearylalkohol, Cetylpalmitat, Kokosnußglyceride, (3)

Cutina FS 45: Palmitin/Stearinsäure-Mischung, (3)

Cutina MD: Glycerinstearat, (3)

Dehymuls HR E 7: ethoxyliertes hydriertes Ricinusöl, (3)

Dehyquart: Cetyltrimethylammoniumchlorid, (3)

D-Panthenol: D-Panthenol, (6)

Eumulgin B 1: Cetyl-/Stearylalkohol, verethert mit 12 Mol Ethylenoxid, (3)

Eumulgin B 2: Cetyl-/Stearylalkohol, verethert mit 20 Mol Ethylenoxid, (3)

Eutanol G: 2-Octyldodecanol, (3)

Genapol LRO fl.: Natriumlaurylsulfat,(10)

Glycerin 86 %: Glycerin, (3) Isopropylisostearat: Isopropylisostearat,(7)

Jojobaöl: Jojobaöl, (8)

Lameform TGI: Triglycerindiisostearat, (3)

Lamepon S: Eiweiß/Kokosfettsäure-Kondensat, Kaliumsalz, (3)

Lanette O: Cetyl-/Stearylalkohol-Mischung, (3)

Lunacera LBN: Octylstearat, Octylcocoat, Ceresin, PEG-8 Bienenwachs, Carnaubawachs, Candelillawachs, (15)

Luviskol K 30 Vinylpyrrolidon Copolymer, (6)

Myritol 318: Capryl-/Caprin-triglycerid, (3)

Neo Heliopan, Typ AV: p-Methoxyzimtsäureisooctylester, (1)

Neo Heliopan, Typ BB: 2-Hydroxy-4-methoxybenzophenon, (1)

Neo Heliopan,Typ Hydro: Phenylbenzimidazolsulfonsäure, (1)

Neo Heliopan, Typ MBC: 4-Methylbenzyliden-campher, (1)

Neo Heliopan, Typ OS : Octylsalicylat, (1)

Neo Heliopan, Typ 303 : Octocrylen, (1)

Neutrol TE: Tetrahydroxypropyl Ethylendiamin, (6)

Phenonip: Gemisch von p-Hydroxybenzoesäureestern, (6)

Permulgin: Wachs, (13)

Polymer JR 400: Polyquaternium-10,(11)

Rewopal PIB 1000: Polyisobutylen, (17)

Solbrol M: p-Hydroxybenzoesäuremethylester, (5)

Solbrol P: p-Hydroxybenzoesäurepropylester, (5)

Tegin M: Glycerinstearat, (8)

Tagat S: Polyoxyethylenglycerinmonostearat, (8)

Texapon MG 3: Magnesiumlaurylsulfat/Dinatriumlaurylsulfosuccinat, (3)

Tocopherolöl CLR: natürliches Vitamin E in Sojaöl,(16)

Trilon B fl.: Tetranatrium-ethylendiamintetraessigsäure, (6)

Vaseline Typ "Merkur 1546": Petroleum, (14)

Veegum Ultra: Magnesiumaluminiumsilikat, (12)

Zinc Oxide neutral H&R: Zinkoxid neutral, (1)

Bezugsquellen:

(1) Haarmann und Reimer GmbH; Holzminden

(2) B.F. Goodrich Comp., Neuss

(3) Henkel KGaA, Düsseldorf

(4) ICI Speciality Chemicals, Frankfurt

(5) Bayer AG, Leverkusen

(6) BASF, Ludwigshafen

(7) Gattefosse, Saint-Priest Cedex

(8) Goldschmidt AG, Essen

(9) Nipa Lab. Ltd., Pontypridd Mid Glam, Wales, GB

(10) Hoechst AG, Frankfurt

(11) Nordmann & Raßmann GmbH & Co, Hamburg

(12) R.T.Vanderbilt Comp. Inc., Norwalk, USA

(13) Koster Keunen Holland B.V., Bladel, NL

(14) Deutsche Texaco AG, Hamburg

(15) Fuller GmbH, Lüneburg

(16) Richter GmbH, Berlin

(17) Rewo, Chemische Fabrik, Steinau

## Patentansprüche

1. Natürlicher p-Methoxyzimtsäureisoamylester.

2. Verfahren zur Herstellung von höheren p-Methoxyzimtsäurealkylestern durch enzymatische Umesterung von natürlichem p-Methoxyzimtsäure-ethylester mit natürlichen höheren Alkoholen.

3. Verwendung von p-Methoxyzimtsäureisoamylester nach Anspruch 1 und von nach Verfahren gemäß Anspruch 2 erhältlichen Verbindungen als UV-Lichtschutzfilter .

4. Verwendung von natürlichen p-Methoxyzimtsäureethylester enthaltendem Kaempferia-galanga-Extrakt als UV-Lichtschutzfilter.